(19) Europäisches Patentamt
European Patent Office
Office européen des brevets

(11) **EP 3 583 893 B1**

(12) **EUROPEAN PATENT SPECIFICATION**

(45) Date of publication and mention
of the grant of the patent:
**06.04.2022 Bulletin 2022/14**

(21) Application number: **18754489.5**

(22) Date of filing: **09.02.2018**

(51) International Patent Classification (IPC):
**A61B 5/026** (2006.01)    **G01N 21/49** (2006.01)
**A61B 5/02** (2006.01)    **A61B 5/00** (2006.01)

(52) Cooperative Patent Classification (CPC):
**A61B 5/0261; A61B 5/0059; A61B 5/02007;**
**G01N 2021/479**

(86) International application number:
**PCT/JP2018/004512**

(87) International publication number:
**WO 2018/151022 (23.08.2018 Gazette 2018/34)**

(54) **SCATTERED BODY MEASUREMENT DEVICE AND SCATTERED BODY MEASUREMENT METHOD**

STREUKÖRPERMESSVORRICHTUNG UND STREUKÖRPERMESSVERFAHREN

DISPOSITIF DE MESURE DE CORPS DISSÉMINÉ ET PROCÉDÉ DE MESURE DE CORPS DISSÉMINÉ

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **14.02.2017 JP 2017024692**

(43) Date of publication of application:
**25.12.2019 Bulletin 2019/52**

(60) Divisional application:
**21214840.7**

(73) Proprietor: **Medical Photonics Co., Ltd.**
**Sapporo-shi, Hokkaido 001-0021 (JP)**

(72) Inventor: **IINAGA, Kazuya**
**Sapporo-shi**
**Hokkaido 001-0021 (JP)**

(74) Representative: **J A Kemp LLP**
**80 Turnmill Street**
**London EC1M 5QU (GB)**

(56) References cited:
**WO-A1-2016/117106    JP-A- 2006 102 171**
**JP-A- 2007 105 331    JP-A- 2007 125 144**
**JP-A- 2015 226 754    JP-A- 2016 007 503**
**JP-B1- 6 029 128    US-A1- 2007 100 245**
**US-A1- 2014 049 775**

• **KAZUYA IINAGA ET AL: "Attempt for noninvasive evaluation of in vivo triglyceride in blood", 2001 CONFERENCE PROCEEDINGS OF THE 23RD ANNUAL INTERNATIONAL CONFERENCE OF THE IEEE ENGINEERING IN MEDICINE AND BIOLOGY SOCIETY, vol. 2013, 1 July 2013 (2013-07-01), pages 1214-1217, XP055605275, IEEE PISCATAWAY, NJ, USA, ISSN: 1094-687X, DOI: 10.1109/EMBC.2013.6609725**

**Description**

Technical Field

The present invention relates to a scatterer measuring apparatus and a scatterer measuring method.

Background Art

Attention has been directed to postprandial hyperlipidemia as a risk factor of the arteriosclerosis. There has been a report stating that an increase in neutral lipid concentration in a non-hunger state increases the risk of development of an event of coronary artery diseases.

WO 2016/117106 A1 discloses a blood-vessel recognition device and surgical treatment device. JP 6 029128 B1 discloses a blood lipid concentration measuring device and operation method thereof.

To diagnose the postprandial hyperlipidemia, it is necessary to observe a change in in-blood lipid concentration for 6 to 8 hours after meals. That is, to measure the state of postprandial hyperlipemia, it is necessary to place a subject under restraint for 6 to 8 hours and collect the blood multiple times. The diagnosis of the postprandial hyperlipidemia is therefore no better than clinical studies, and diagnosing the postprandial hyperlipidemia at a clinical site is not practical.

Patent Literature 1 discloses an approach to a solution of the problem described above. According to the approach disclosed in Patent Literature 1, the noninvasive lipid measurement can eliminate the blood collection. The in-blood lipid can therefore be measured not only in a medical institution but at home. Allowing instantaneous data acquisition allows temporally continuous in-blood lipid measurement.

Citation List

Patent Literature

Patent Literature 1: International Publication No. WO 2014/087825

Summary of Invention

Technical Problem

In the noninvasive lipid measuring approach shown in Patent Literature 1, however, the measurement is performed on the arteries, capillaries, veins, and other blood vessels irrespective of the type of blood vessel.

On the other hand, the blood flows sequentially "from the arteries to the capillaries," and "from the capillaries to the veins," and it is known that lipid in particular is metabolized also in the arteries and capillaries. That is, knowledge on the state of the blood in each blood vessel allows a detailed study on the metabolism.

The present invention has been made to solve the challenge in the related art, and an object of the present invention is to provide an apparatus and a method that allow detection of scatterer information.

Solution to Problem

A scatterer measuring apparatus according to the present invention is defined by the appended claim 1.

A scatterer measuring method according to the present invention is defined by the appended claim 6.

Also disclosed is a scatterer measuring apparatus communicably connected to a user apparatus including an irradiator that radiates phase-aligned light having a predetermined light intensity to a predetermined site of a living body from outside the living body toward an interior of the living body, a light intensity detector that detects a light intensity distribution of light emitted from the living body, and a communicator that transmits the light intensity distribution detected by the light intensity detector, and the scatterer measuring apparatus includes a controller that calculates a blood flow rate based on a temporal change in the light intensity distribution transmitted from the user apparatus and calculates scatterer concentration in the living body based on the blood flow rate.

Advantageous Effect of Invention

The scatterer measuring apparatus and the scatterer measuring method according to the present invention allow detection of scatterer information.

Brief Description of Drawings

[Figure 1] Figure 1 shows a configuration of a scatterer measuring apparatus according to an embodiment.
[Figure 2] Figure 2 shows light scattered by in-blood lipid.
[Figure 3] Figure 3 is a block diagram of the scatterer measuring apparatus according to the embodiment.
[Figure 4] Figure 4 is a flowchart of a scatterer measuring method according to the embodiment.
[Figure 5] Figure 5 shows a configuration of a scatterer measuring system according to the embodiment.
[Figure 6] Figure 6 is a block diagram of the scatterer measuring apparatus according to the embodiment.
[Figure 7] Figure 7 shows bright and dark portions created by optical interference.
[Figure 8A] Figure 8A shows a measurement result in a case where in-blood TG is changed.
[Figure 8B] Figure 8B shows direct comparison between a flow rate and TG after lipid loading.
[Figure 9] Figure 9 shows a relationship between a particle diameter and the flow rate.
[Figure 10] Figure 10 shows a difference in optical path of light.
[Figure 11] Figure 11 shows the optical interference.
[Figure 12] Figure 12 shows another configuration of the scatterer measuring apparatus according to the embodiment.
[Figure 13] Figure 13 shows still another configuration of the scatterer measuring apparatus according to the embodiment.

Description of Embodiments

A scatterer measuring apparatus according to an embodiment of the present invention and a method for operating the same will be described below in detail with reference to the drawings. The following description will be primarily made of detection of in-blood lipid as an example of the scatterer, but not limited to this, and the embodiment is also applicable to general in-blood scatterers.

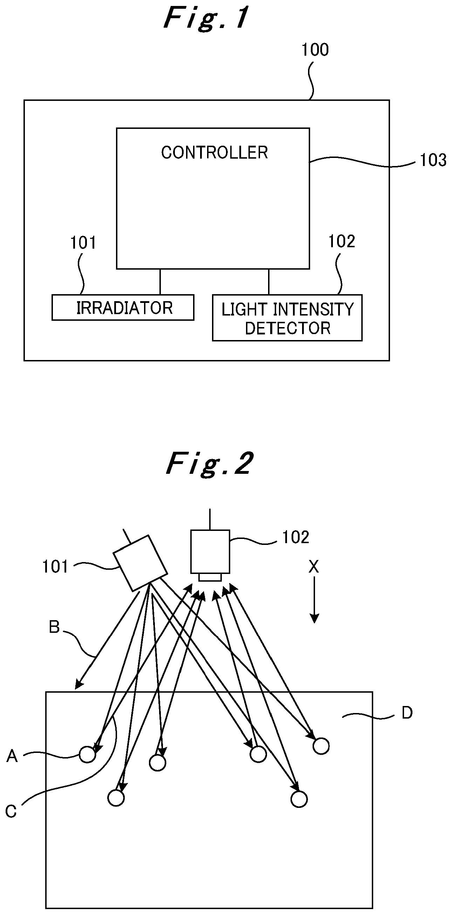

Figure 1 shows a configuration of the scatterer measuring apparatus according to the embodiment.

A scatterer measuring apparatus 100 according to the embodiment includes an irradiator 101, which radiates phase-aligned light to a predetermined site of a living body from outside the living body toward the interior of the living body, a light intensity detector 102, which receives light emitted from the living body and detects a two-dimensional light intensity distribution of light from scatterers contained in the living body based on optical interference, and a controller 103, which calculates a blood flow rate based on a temporal change in the two-dimensional light intensity distribution detected by the light intensity detector 102 and calculates scatterer concentration based on the blood flow rate, as shown in Figure 1.

The irradiator 101 includes a light source for radiating the phase-aligned light to a predetermined radiation position on the predetermined site of the living body from outside the living body toward the interior of the living body, as shown in Figure 1. In the embodiment, the phase-aligned light is laser light. The irradiator 101 includes a wide-angle lens. The irradiator 101 uses the wide-angle lens to increase the diameter of the laser light to a value ranging from 20 to 30 cm. The value to which the diameter is increased is not limited to the values described above. In the present embodiment, a wide-angle lens is used to expand the laser light, but not limited to this, and the predetermined site of the living body may instead be scanned with the laser light. The irradiator 101 radiates near-infrared light onto the entire skin surface to detect information on lipid in the blood in the capillaries.

Laser light is used as the phase-aligned light in the embodiment, and the light source may output any phase-aligned light. For example, as a light source that outputs phase-aligned light, an LED or any other light source with a pinhole, a lens, or a tunnel-shaped tube may be used.

The irradiator 101 in the embodiment can adjust the wavelength of the radiated light. The irradiator 101 can adjust the wavelength range in such a way that the wavelength range does not fall within the range of the wavelengths at which the light is absorbed by inorganic substances of the blood plasma. The irradiator 101 can further adjust the wavelength range in such a way that the wavelength range does not fall within the range of the wavelengths at which the light is absorbed by cell components in the blood. The cell components in the blood are formed of the red blood cells, white blood cells, and platelets in the blood. The inorganic substances of the blood plasma are formed of water and electrolytes in the blood.

The range of the wavelength of the light radiated by the irradiator 101 is preferably shorter than or equal to about 1400 nm and from about 1500 to 1860 nm in consideration of the range of the wavelengths at which the light is absorbed by the inorganic substances of the blood plasma. Further, the range of the wavelength of the light radiated by the irradiator 101 is more preferably from about 580 to 1400 nm and from about 1500 to 1860 nm in consideration of the range of the wavelengths at which the light is absorbed by the cell components in the blood.

The thus set wavelength range used by the irradiator 101 suppresses the influence of the inorganic substances of the blood plasma on absorption of light to be detected by the light intensity detector 102, which will be described later, and the influence of the cell components in the blood on the light absorption. In the thus set wavelength range, no absorption large enough to identify a substance is present, whereby optical energy loss due to the absorption is negligibly small. The light in the blood therefore propagates over a large distance when scattered by lipid in the blood and exits out of the living body.

The irradiator 101 in the embodiment can arbitrarily adjust the time length, for example, for which continuous light or pulsed light is radiated. The irradiator 101 can arbitrarily modulate the intensity or phase of the radiated light.

The irradiator 101 may be formed of a light source having a fixed wavelength. The irradiator 101 may instead be formed of a plurality of light sources that output light fluxes having different wavelengths or the combination of light fluxes having a plurality of wavelengths.

The light intensity detector 102 receives light emitted out of the living body and detects the two-dimensional light intensity distribution of the received light.

Figure 2 shows light scattered by in-blood lipid. The light (B in Figure 2) radiated from the irradiator 101 to a radiation position on the surface of a living body D arrives at the depth where lipid, such as lipoprotein, is present and is then reflected off in-blood lipid (A in Figure 2) in the living body D, as shown in Figure 2. Further, the radiated light is scattered by the in-blood lipid, and resultant back-scattered light (C in Figure 2) is then emitted from the living body. The light intensity detector 102 detects the light intensity of the back-scattered light C.

In Figure 2, the distance from the front end of the irradiator 101 to the surface of the living body D is 30 cm. The distance is not, however, limited to the value described above.

Lipoprotein, which is the target under measurement, has a spherical structure covered with apoprotein and other substances. Lipoprotein is present in the form of a solid-like state in the blood. Lipoprotein has the property of reflecting light. In particular, cylomicron (CM), VLDL, and other substances each having a large particle diameter and specific gravity contain a large amount of triglyceride (TG) and they are more likely to scatter light. The light intensity detected by the light intensity detector 102 is affected by the light scatted by lipoprotein.

The light intensity detector 102 may be a CCD or CMOS device or any other light receiving device. The light intensity detector 102 may instead be formed of light receiving devices arranged in an array or in a concentric form. To reduce the number of light receiving devices, the light receiving devices may be arranged in the form of a cross or a letter V around a radiation position E.

The laser light radiated by the irradiator 101 is characterized by travel straightness. Therefore, when a living body or any other object is irradiated with the laser light, differences in the optical path length cause interference, resulting in a light intensity distribution. As a result, an image detected by the light intensity detector 102 shows optically bright and dark portions in the form of a mottled pattern.

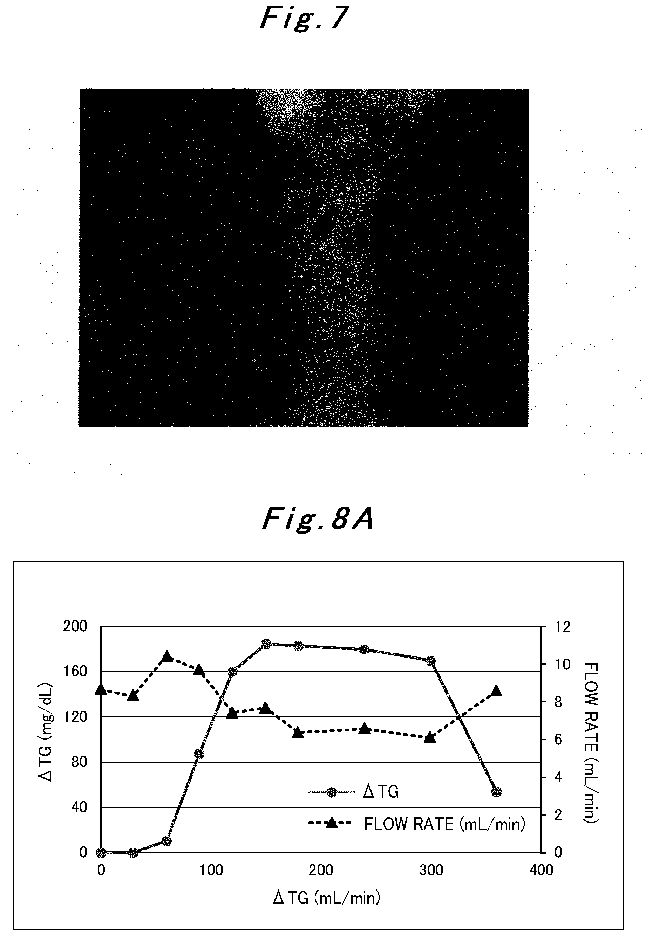

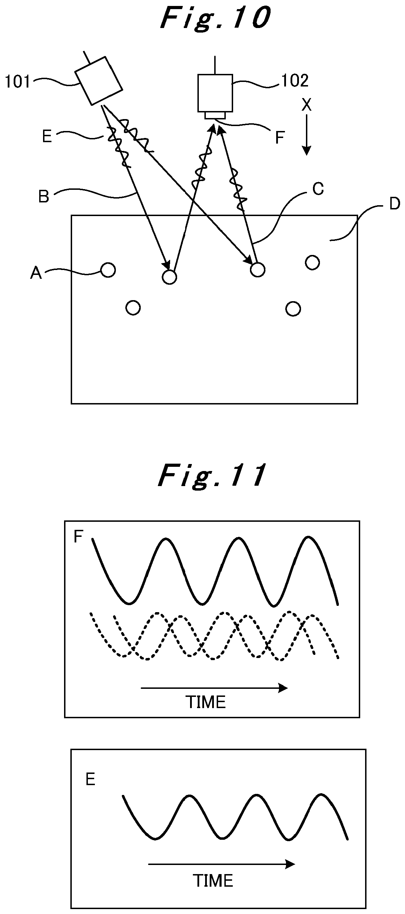

For example, the irradiator 101 outputs light fluxes having the same frequency (E in Figure 11), as shown in Figure 10, and the light fluxes interfere with each other as indicated by F in Figure 11 on the light receiving surface of the light intensity detector 102 due to the difference in the optical path to in-blood lipid and the distance from a scatterer to the light reception surface. An image acquired by the light intensity detector 102 shows bright and dark portions, such as those shown in Figure 7. The magnitude of the intensity of the received light changes in accordance with the optically bright and dark portions as the subject changes with time. The light intensity detector 102 captures the magnitude of the intensity of the received light in a two-dimensional form.

It is believed that the present approach allows observation of the optical interference up to about 1 mm below the skin, and the resultant blood information represents information on the capillaries.

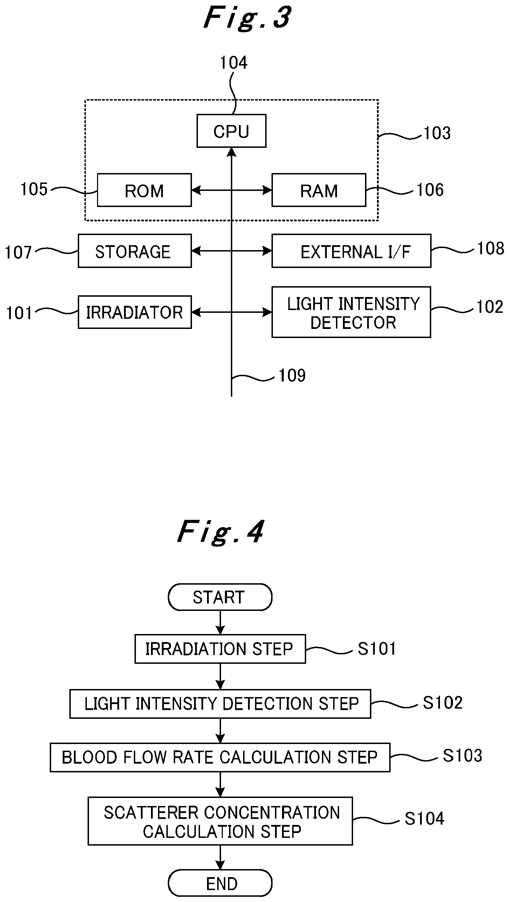

The configuration of a control system of the scatterer measuring apparatus 100 will next be described. Figure 3 is a block diagram of the scatterer measuring apparatus 100 according to the embodiment.

A central processing unit (CPU) 104, a read only memory (ROM) 105, a random access memory(RAM) 106, a storage 107, an external interface (I/F) 108, the irradiator 101, and the light intensity detector 102 are connected to each other via a system bus 109. The CPU 104, the ROM 105, and the RAM 106 form the controller 103.

The ROM 105 stores in advance a program executed by the CPU 104 and thresholds used by the CPU 104.

The RAM 106 has an area where the program executed by the CPU 104 is developed, a variety of memory areas, such as a work area where the program processes data, and other areas.

The storage 107 stores data prepared in advance and representing the relationship between the blood flow rate and the scatter concentration in a living body. The storage 107 may be an internal memory that stores information in a nonvolatile manner, such as a hard disk drive (HDD), a flash memory, and a solid-state drive (SSD) .

The external I/F 108 is an interface for communication with an external apparatus, for example, a client terminal (PC). The external I/F 108 only needs to be an interface that performs data communication with an external apparatus and may, for example, be an instrument (such as USB memory) locally connected to the external apparatus or a network interface for communication via a network.

The controller 103 calculates the blood flow rate based on a temporal change in the two-dimensional light intensity distribution detected by the light intensity detector 102.

To perform the measurement by using a CCD as the light intensity detector 102, data is acquired on a pixel basis. The laser speckles or the mottled pattern created by the interference (speckle pattern) does not move unless the scatterers move. In other words, when the scatterers move, the speckle pattern moves. The speckle pattern is expressed by the product of the amount of scatterers and how fast the scatterers move.

For example, in the measurement of a human skin, scatterers that move in a short period are only blood, and what is measured is the blood flow. However, since movement of a portion under measurement affects the speckle pattern, the subject under measurement needs to keep stationary upon measurement.

The controller 103 binarizes an image acquired by the light intensity detector 102 and calculates the blood flow rate from the light intensities for 10 seconds at each pixel value of the image and the standard deviation of the light intensities.

The controller 103 calculates the in-blood scatterer concentration based on the calculated blood flow rate.

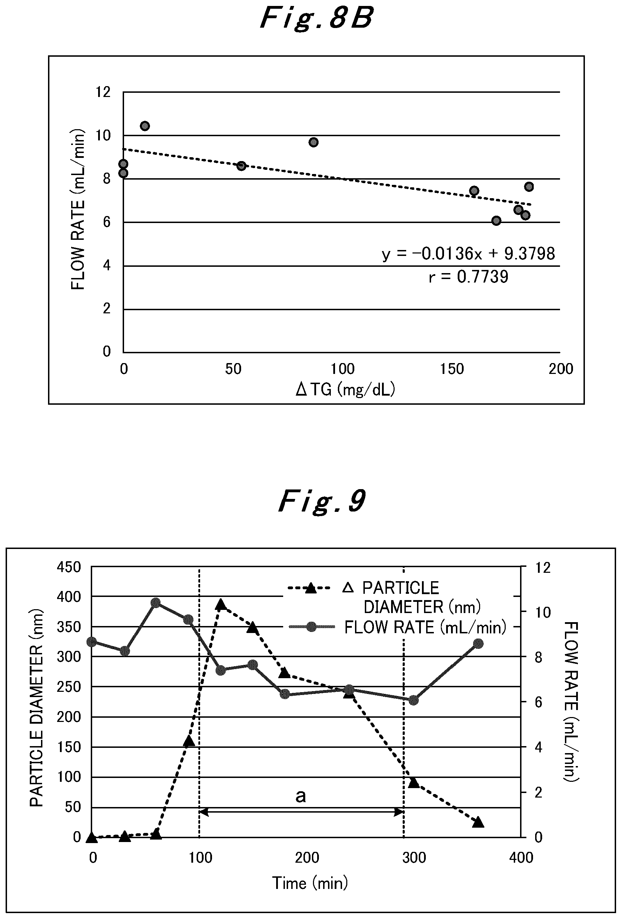

Figure 8 shows a result of the measurement in a case where lipid is loaded into a subject to change the in-blood TG. Figure 8A shows a temporal change in the blood flow rate and a temporal change in TG after the lipid loading. Figure 8A shows that the two temporal changes are inversely correlated to each other as TG increases or decreases. Figure 8B shows direct comparison between the blood flow rate and TG after the lipid loading. Figure 8B shows a close correlation having a correlation coefficient of 0.7739.

To examine measured lipid metabolism, blood serum was separated from the blood collected after the lipid loading, and dynamic light scattering (DLS) was then used to measure the diameter of particles in the blood serum. To focus on the size of the lipid particles after meals, particle variation due to lipid was obtained by subtracting the particle diameter at the time of hunger from the measured diameter. Figure 9 shows the result of the particle diameter measurement and the flow rate.

As a result, the flow rate decreased in period "a" in Figure 9 after large lipid particles appeared, and the flow rate recovered to a value roughly equal to the value at the time of hunger as the large particles disappeared.

CM (cylomicron: particle diameter ranging from 80 to 1000 nm), which is a large lipid particle that appears after meals, is anchored by LPL present at the surface of the capillaries and moves TG in CM into the tissue. The lipid particles measured in Figure 8 can be determined based on the particle diameter thereof to be CM. That is, it is inferred that CM anchored in the capillaries blocked the blood flow to cause the decrease in the flow rate. It is therefore believed that the blood flow was restored after CM was metabolized. Further, focusing on the result in the vicinity of 300 min shows that there is a time lag between the recovery of the blood flow and the disappearance of CM, but it is shown that the particle diameter of CM is greater than or equal to 80 nm and CM is still present in the blood at 300 min. As described above, lipid in the capillaries can be measured. Comparison of results of the blood between the capillaries and the veins allows more detailed observation of the lipid metabolism.

Further, LPL activity is related to insulin concentration, and insulin resistance and the like can be examined, for example, from the CM metabolism speed.

Moreover, the CM metabolization period can also be used as an index representing the presence of abnormal lipid, such as chylomicron remnant (CM-R).

Blocked blood flow that accompanies an increase in lipid can also be checked from the principle of the measurement. First, the blood flow rate is expressed by the following expression:

```
Blood flow rate = volume (concentration) × speed
```

In a case where the number of scatterers increases, the blood flow rate increases based on the expression described above. However, the result of the measurement shows that the flow speed relatively decreases also from the fact that the blood flow rate decreases as the lipid concentration increases.

Instead, the controller 103 may calculate a scattering coefficient from the blood flow rate and then calculate the lipid concentration (scatterer concentration). At a clinical site, the concentration and the turbidity are synonymous with each other in some cases, and the concentration in the present invention includes the turbidity. The scatterer concentration calculator 104 can therefore calculate not only the concentration but the number of particles per unit amount, the formazin turbidity, or the scattering coefficient as the results of the calculation.

A scatterer measuring method according to the embodiment will next be described. Figure 4 is a flowchart of the scatterer measuring method according to the embodiment.

In a radiation step (S101), the irradiator 101 radiates phase-aligned light (laser light, for example) to a radiation position on a living body.

In a light intensity detection step (S102), the light intensity detector 102 receives light emitted from the living body and detects a two-dimensional light intensity distribution of the light fluxes that come from the scatterers contained in the living body and interfere with each other. The light intensity distribution detected in the light intensity detection step is sent to a blood flow rate calculation step.

In the blood flow rate calculation step (S103), the controller 103 calculates the blood flow rate based on a temporal change in the light intensity distribution. The calculated blood flow rate is sent to a scatterer concentration calculation step.

In the scatterer concentration calculation step (S104), the controller 103 calculates the in-blood scatterer concentration based on the blood flow rate. In the scatterer concentration calculation step, the lipid concentration (scatterer concentration) may be calculated after the scattering coefficient is calculated from the blood flow rate.

As described above, the scatterer measuring apparatus and the scatterer measuring method according to the present embodiment allow calculation of the scatterer concentration by acquiring a temporal change in the light intensity distribution of the light emitted from a living body.

A scatterer measuring apparatus according to another embodiment of the present invention will next be described. Some portions of a configuration of the scatterer measuring apparatus according to the other embodiment of the present invention are the same as those of the configuration of the scatterer measuring apparatus according to the embodiment described above, and different portions will therefore be primarily described.

In the embodiment described above, the configuration in which the irradiator 101, the light intensity detector 102, and the controller are integrated with one another has been presented by way of example, but not limited to this. The irradiator 101, the light intensity detector 102, and the controller may be configured as a system in which the irradiator 101 and the light intensity detector 102 are configured as a user apparatus and the controller is provided in a server apparatus connected to the user apparatus.

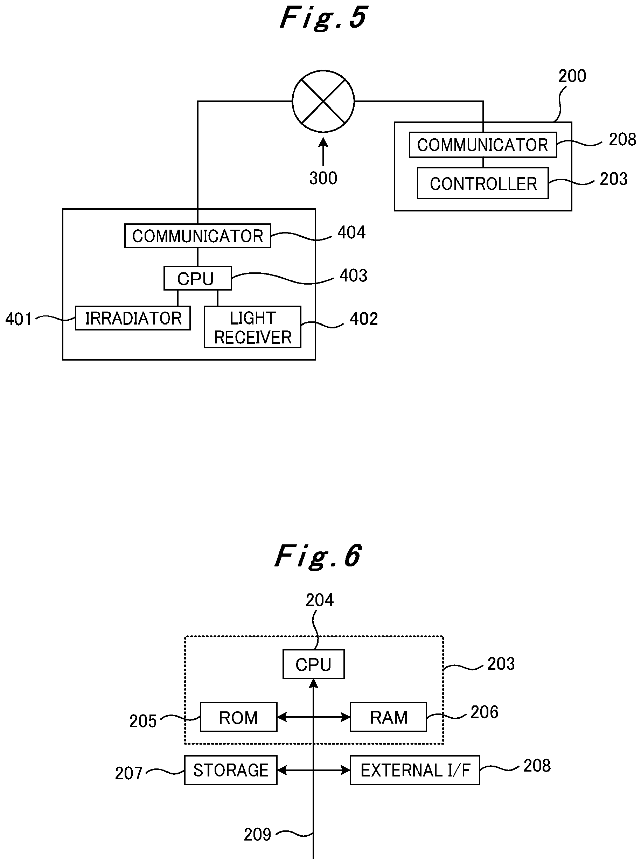

Figure 5 is a block diagram showing the configuration of a scatterer measuring system according to the embodiment.

The system includes a scatterer measuring apparatus 200, an access point 300, and a user apparatus 400.

The scatterer measuring apparatus 200 is an apparatus for calculating the scatterer concentration by carrying out a predetermined process based on light intensity transmitted from the user apparatus 400. Specifically, the scatterer measuring apparatus 200 is a personal computer, or depending on the number of apparatuses and the amount of data to be transmitted and received, the scatterer measuring apparatus 200 is a server apparatus as appropriate.

The user apparatus 400 is an apparatus possessed by a user and is a standalone apparatus in some cases or is incorporated in a smartphone, a mobile phone, or a wristwatch in other cases. A camera, illumination, a communication function, and the like provided in a smartphone or a mobile phone may be used as an irradiator 401, a light intensity detector 402, and a communicator 404.

The user apparatus 400 includes the irradiator 402, which radiates phase-aligned light (laser light, for example), the light intensity detector 402, and the communicator 404. The communicator 404 transmits the light intensity detected by the light intensity detector 401. The functions and operations of the irradiator 401 and the light intensity detector 402 have been described above.

The scatterer measuring apparatus 200 includes a communicator 204 and a controller 203. The communicator 204 receives the light intensity transmitted from the communicator 404 via the access point 300 and transmits the light intensity to the controller 203.

A configuration of a control system of the scatterer measuring apparatus 200 will next be described. Figure 6 is a block diagram of the scatterer measuring apparatus 200 according to the embodiment. A central processing unit (CPU) 204, a read only memory (ROM) 205, a random access memory (RAM) 206, a storage 207, a communicator (external interface (I/F)) 208 are connected to each other via a system bus 209. The CPU 204, the ROM 205, and the RAM 206 form the controller 203.

The ROM 205 stores in advance a program executed by the CPU 204 and thresholds used by the CPU 204.

The RAM 206 has an area where the program executed by the CPU 204 is developed, a variety of memory areas, such as a work area where the program processes data, and other areas.

The storage 207 stores data prepared in advance on appropriate numerical ranges of static and dynamic parameters. The storage 207 may be an internal memory that stores information in a nonvolatile manner, such as a hard disk drive (HDD), a flash memory, and a solid-state drive (SSD).

The communicator (external I/F) 208 is an interface for communication with an external apparatus, for example, a client terminal (PC). The external I/F 208 only needs to be an interface that performs data communication with the external apparatus and may, for example, be an instrument (such as USB memory) locally connected to the external apparatus or a network interface for communication via a network. The functions and operations of the controller 203 have been described above.

In the embodiment, the light intensity is transmitted from the user apparatus 400 to the scatterer measuring apparatus 200 via the access point 300, but not limited to this, and the user apparatus 400 and the scatterer measuring apparatus 200 may be directly connected to each other via no access point, and the scatterer measuring apparatus 200 may transmit the light intensity over wired communication, wireless communication, or any other means.

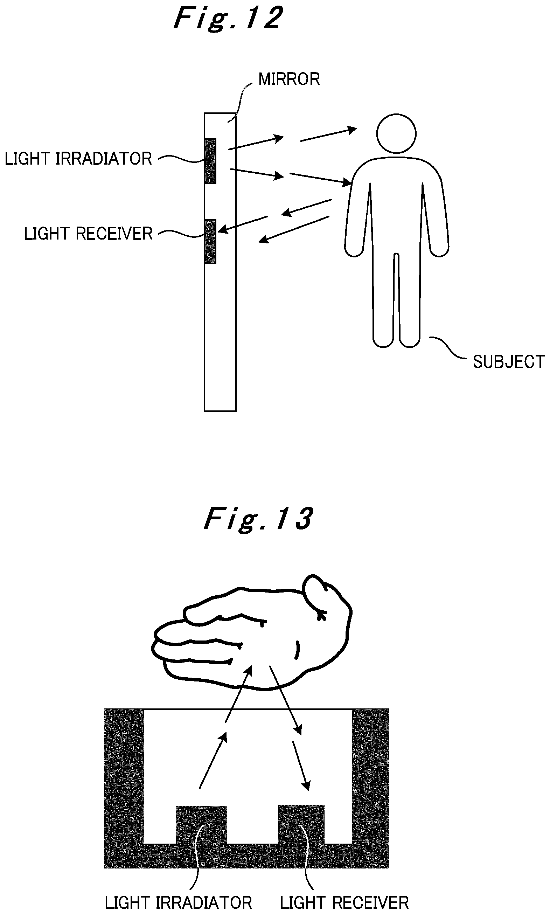

The measurement may, for example, be so performed that the measurement burden is reduced in the daily life, as shown in Figure 12, by providing a mirror with the irradiator and the light intensity detector, radiating the laser light from the irradiator (light irradiator in Figure 12) to a subject, for example, from the side behind the mirror, and installing the light intensity detector (light receiver in Figure 12), for example, on the side behind the mirror to allow the user to perform the measurement when the user washes the face. Further, since the user himself/herself only needs to be projected on a surface, the irradiator and the light intensity detector may be incorporated in a palmtop mirror, a smartphone, a tablet (collectively called a self-projection apparatus).

Instead, as shown in Figure 13, the irradiator (light irradiator in Figure 13) and the light intensity detector (light receiver in Figure 13) may be disposed at the bottom of a box-shaped body having a concave crosssection, and measurement may be performed by a user placing a hand over the box-shaped body.

Reference Sings list

100   Scatterer measuring apparatus
101   Irradiator
102   Light intensity detector
103   Blood flow rate calculator
104   Scatterer concentration calculator

**Claims**

1.  A scatterer measuring apparatus (100), comprising:

    an irradiator (101) configured to radiate phase-aligned light at a predetermined light intensity suitable for radiating the light to a predetermined site of a living body (D) from outside the living body toward an interior of the living body; wherein the apparatus further comprises:

    a light intensity detector (102) configured to detect a two-dimensional light intensity distribution of light emitted from the living body; and
    a controller (103) configured to calculate a blood flow rate based on a temporal change in the two-dimensional light intensity distribution and to calculate scatterer concentration in the living body based on the blood flow rate,
    wherein the irradiator includes optical means for expanding the phase-aligned light.

2.  The scatterer measuring apparatus according to claim 1, wherein the phase-aligned light is laser light.

3.  The scatterer measuring apparatus according to any of claims 1 to 2, wherein the controller is configured to calculate a scattering coefficient from the blood flow rate and then to calculate the scatterer concentration.

4.  The scatterer measuring apparatus according to any of claims 1 to 3, further comprising a mirror or a self-projection apparatus, wherein the irradiator and the light intensity detector are provided in the mirror or the self-projection apparatus.

5.  The scatterer measuring apparatus according to any of claims 1 to 3, further comprising a box-shaped body, wherein the irradiator and the light intensity detector are provided at a bottom of the box-shaped body.

6.  A scatterer measuring method, comprising:

    a radiation step of radiating phase-aligned light having a predetermined light intensity to a predetermined site of a living body (D) from outside the living body toward an interior of the living body;
    a light intensity detection step of detecting a two-dimensional light intensity distribution of light emitted from the living body;
    a blood flow rate calculation step of calculating a blood flow rate based on a temporal change in the two-dimensional light intensity distribution; and
    a scatterer concentration calculation step of calculating scatterer concentration in the living body based on the blood flow rate,
    wherein the phase-aligned light is expanded in the radiation step.

**7.** The scatterer measuring method according to claim 6, wherein the phase-aligned light is laser light.

**8.** The scatterer measuring method according to any of claims 6 to 7, wherein the scatterer concentration calculation step includes calculating a scattering coefficient from the blood flow rate and then calculating the scatterer concentration.

**9.** The scatterer measuring method according to any of claims 6 to 8, wherein the scatterer is lipid in a capillary.

**Patentansprüche**

**1.** Streuermesseinrichtung (100), umfassend:

ein Bestrahlungsgerät (101), das konfiguriert ist, um phasenausgerichtetes Licht mit einer vorbestimmten Lichtintensität auszustrahlen, die geeignet ist, um Licht zu einer vorbestimmten Stelle eines lebenden Körpers (D) von dem Äußeren des lebenden Körpers zu einem Inneren des lebenden Körpers auszustrahlen;
wobei die Einrichtung ferner umfasst:

einen Lichtintensitätsdetektor (102), der konfiguriert ist, um eine zweidimensionale Lichtintensitätsverteilung von Licht, das von dem lebenden Körper emittiert wird, zu erfassen; und
eine Steuervorrichtung (103), die konfiguriert ist, um eine Blutdurchflussrate auf der Grundlage einer zeitlichen Änderung der zweidimensionalen Lichtintensitätsverteilung zu berechnen und eine Streuerkonzentration in dem lebenden Körper auf der Grundlage der Blutdurchflussrate zu berechnen,
wobei das Bestrahlungsgerät optische Mittel zum Ausdehnen des phasenausgerichteten Lichts beinhaltet.

**2.** Streuermesseinrichtung nach Anspruch 1, wobei das phasenausgerichtete Licht ein Laserlicht ist.

**3.** Streuermesseinrichtung nach einem der Ansprüche 1 bis 2, wobei die Steuervorrichtung konfiguriert ist, um einen Streukoeffizienten von der Blutdurchflussrate zu berechnen und dann die Streuerkonzentration zu berechnen.

**4.** Streuermesseinrichtung nach einem der Ansprüche 1 bis 3, ferner umfassend einen Spiegel oder eine Selbstprojektionseinrichtung, wobei das Bestrahlungsgerät und der Lichtintensitätsdetektor in dem Spiegel oder der Selbstprojektionseinrichtung bereitgestellt sind.

**5.** Streuermesseinrichtung nach einem der Ansprüche 1 bis 3, ferner umfassend einen kastenförmigen Körper, wobei das Bestrahlungsgerät und der Lichtintensitätsdetektor auf einem Boden des kastenförmigen Körpers bereitgestellt sind.

**6.** Streuermessverfahren, umfassend:

einen Ausstrahlungsschritt des Ausstrahlens von phasenausgerichtetem Licht mit einer vorbestimmten Lichtintensität auf eine vorbestimmte Stelle eines lebenden Körpers (D) von dem Äußeren des lebenden Körpers zu einem Inneren des lebenden Körpers;
einen Lichterfassungsschritt des Erfassens einer zweidimensionalen Lichtintensitätsverteilung von Licht, das von dem lebenden Körper emittiert wird;
einen Blutdurchflussratenberechnungsschritt des Berechnens einer Blutdurchflussrate auf der Grundlage einer zeitlichen Änderung der zweidimensionalen Lichtintensitätsverteilung; und
einen Streuerkonzentrationsberechnungsschritt des Berechnens einer Streuerkonzentration in dem lebenden Körper auf der Grundlage der Blutdurchflussrate,
wobei das phasenausgerichtete Licht in dem Ausstrahlungsschritt ausgedehnt wird.

**7.** Streuermessverfahren nach Anspruch 6, wobei das phasenausgerichtete Licht ein Laserlicht ist.

**8.** Streuermessverfahren nach einem der Ansprüche 6 bis 7, wobei der Streuerkonzentrationsberechnungsschritt das Berechnen eines Streuerkoeffizienten von der Blutdurchflussrate und dann das Berechnen der Streuerkonzentration beinhaltet.

**9.** Streuermessverfahren nach einem der Ansprüche 6 bis 8, wobei der Streuer ein Lipid in einer Kapillare ist.

**Revendications**

1. Appareil de mesure de diffuseur (100), comprenant :

   un irradiateur (101) configuré pour émettre un rayonnement de lumière à alignement de phase à une intensité lumineuse prédéterminée appropriée pour le rayonnement de la lumière vers un site prédéterminé d'un corps vivant (D) depuis l'extérieur du corps vivant vers un intérieur du corps vivant ;
   dans lequel l'appareil comprend en outre :

   un détecteur d'intensité lumineuse (102) configuré pour détecter une distribution d'intensité lumineuse bidimensionnelle de la lumière émise depuis le corps vivant ; et
   un dispositif de commande (103) configuré pour calculer un débit sanguin sur la base d'un changement temporel de la distribution d'intensité lumineuse bidimensionnelle et pour calculer une concentration de diffuseur dans le corps vivant sur la base du débit sanguin,
   dans lequel l'irradiateur comporte des moyens optiques pour provoquer une expansion de la lumière à alignement de phase.

2. Appareil de mesure de diffuseur selon la revendication 1, dans lequel la lumière à alignement de phase est une lumière laser.

3. Appareil de mesure de diffuseur selon l'une quelconque des revendications 1 à 2, dans lequel le dispositif de commande est configuré pour calculer un coefficient de diffusion à partir du débit sanguin, puis calculer la concentration de diffuseur.

4. Appareil de mesure de diffuseur selon l'une quelconque des revendications 1 à 3, comprenant en outre un miroir ou un appareil d'auto-projection, dans lequel l'irradiateur et le détecteur d'intensité lumineuse sont prévus dans le miroir ou l'appareil d'auto-projection.

5. Appareil de mesure de diffuseur selon l'une quelconque des revendications 1 à 3, comprenant en outre un corps en forme de boîte, dans lequel l'irradiateur et le détecteur d'intensité lumineuse sont prévus au niveau d'un fond du corps en forme de boîte.

6. Procédé de mesure de diffuseur, comprenant :

   une étape de rayonnement consistant à émettre à un rayonnement de lumière à alignement de phase ayant une intensité lumineuse prédéterminée vers un site prédéterminé d'un corps vivant (D) depuis l'extérieur du corps vivant vers un intérieur du corps vivant ;
   une étape de détection d'intensité lumineuse consistant à détecter une distribution d'intensité lumineuse bidimensionnelle de lumière émise depuis le corps vivant ;
   une étape de calcul de débit sanguin consistant à calculer un débit sanguin sur la base d'un changement temporel de la distribution d'intensité lumineuse bidimensionnelle ; et
   une étape de calcul de concentration de diffuseur consistant à calculer une concentration de diffuseur dans le corps vivant sur la base du débit sanguin,
   dans lequel la lumière à alignement de phase est amenée à une expansion dans l'étape de rayonnement.

7. Procédé de mesure de diffuseur selon la revendication 6, dans lequel la lumière à alignement de phase est une lumière laser.

8. Procédé de mesure de diffuseur selon l'une quelconque des revendications 6 à 7, dans lequel l'étape de calcul de concentration de diffuseur comporte le calcul d'un coefficient de diffusion à partir du débit sanguin, puis le calcul de la concentration de diffuseur.

9. Procédé de mesure de diffuseur selon l'une quelconque des revendications 6 à 8, dans lequel le diffuseur est un lipide dans un capillaire.

## *Fig.1*

100

CONTROLLER

103

101

102

IRRADIATOR

LIGHT INTENSITY DETECTOR

## *Fig.2*

102

101

X

B

D

A

C

## *Fig.3*

## *Fig.4*

*Fig.5*

COMMUNICATOR — 208

CONTROLLER — 203

200

300

COMMUNICATOR — 404

CPU — 403

IRRADIATOR — 401

LIGHT RECEIVER — 402

*Fig.6*

204

CPU

203

ROM — 205

RAM — 206

STORAGE — 207

EXTERNAL I/F — 208

209

## *Fig.7*

## *Fig.8A*

## *Fig.8B*

$$y = -0.0136x + 9.3798$$
$$r = 0.7739$$

## *Fig.9*

# Fig.10

# Fig.11

## Fig.12

MIRROR

LIGHT IRRADIATOR

LIGHT RECEIVER

SUBJECT

## Fig.13

LIGHT IRRADIATOR    LIGHT RECEIVER

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016117106 A1
- JP 6029128 B

- WO 2014087825 A